# EUROPEAN PATENT SPECIFICATION

(11) **EP 3 558 120 B1**
(45) Date of publication and mention of the grant of the patent: **04.11.2020**
(21) Application number: 17809349.8
(22) Date of filing: 30.11.2017
(51) Int. Cl.: A61B 5/15, A61B 5/153, A61M 1/14, A61M 25/00, A61M 25/04, A61M 25/01, A61M 25/10

(54) **MIXER SHEATH FOR A VASCULAR CATHETER**
MISCHERHÜLSE FÜR EINEN GEFÄSSKATHETER
GAINE DE MÉLANGEUR DESTINÉE À UN CATHÉTER VASCULAIRE

(30) Priority: 23.12.2016 GB 201622106
(43) Date of publication of application: 30.10.2019
(73) Proprietor: PlaqueTec Ltd., Cambridge, Cambridgeshire CB23 3RE (GB)
(72) Inventor: IVESON, Chris, Rotherham S60 5TZ (GB); BLATCHER, Steve, Cambridge Cambridgeshire CB23 3RE (GB); PEARL, Martin, Cambridge Cambridgeshire CB23 3RE (GB); CORRIGAN, Joe, Cambridge Cambridgeshire CB5 8QG (GB)
(74) Representative: Potter Clarkson
(86) International application number: PCT/GB2017/053611
(87) International publication number: WO 2018/115811

(56) References cited:
- EP-A1- 2 254 637
- US-A1- 2003 073 909
- US-A1- 2012 065 579

## Description

The present disclosure relates to vascular catheters, in particular to vascular catheter systems which are configured to mix blood.

More specifically, an aspect relates to a mixer sheath for a vascular catheter. Further aspects relate to a method of manufacturing such a mixer sheath, a catheter sheath comprising such a mixer sheath, a catheter system comprising such a mixer sheath, a method of deploying a vascular catheter having such a mixer sheath, a method of removing a vascular catheter having such a mixer sheath, a method of deploying a blood mixing element of such a mixer sheath, a method of deploying blood mixing elements of such a mixer sheath, a method of using a catheter system having such a mixer sheath and a method of reversing the deployment of a blood mixing element of such a mixer sheath.

It is sometimes useful to induce mixing of blood flow within a vessel, for example to speed up heating or cooling of blood or to spread a medicament through the circulatory system faster. Mixing of blood from radially outer regions of a vessel towards a centrally located catheter can also be desirable, for example if blood from the boundary layer adjacent the vessel wall needs to be sampled. A blood sampling catheter having blood mixing elements which help to capture boundary layer samples in this way (e.g. to detect biomarkers emitted by vulnerable plaques on the vessel wall) is described in European patent number EP 2 254 637 B1.

As explained in EP 2 254 637 B1, for ease of inserting and removing the catheter, mixing elements are preferably deployable from an inactive state close to the central body of the catheter system, to an active state once in position in a blood vessel. This reduces the risk that mixing elements will traumatise the vessel wall during insertion or removal, potentially causing harm to a patient.

The mixing elements described in EP 2 254 637 B1 can be biased to their deployed, active state, so that they open to their full radial extent (so far as is permitted by the geometry of their location within the vessel) when a constraining outer sheath is withdrawn. The outer sheath can be retracted back over the mixing elements in order to safely remove the catheter.

Document US 2012/0065579 A1 also represents relevant prior art.

It has been found however that manufacturing these kinds of biased mixing elements and attaching them to the central body of the catheter is time-consuming. The manufacturing process must be carefully quality controlled to ensure that the mixing elements are affixed to the catheter central body well enough that the risk of one being stripped from the catheter central body in use (which could be fatal to a patient) is effectively eliminated. Furthermore, multilayer composite structures comprising, for example, polymer film, adhesive and metal foil layers, may act, when housed within the sliding outer sheath, to increase the flexural stiffness of a vascular catheter, potentially limiting its ability to track easily around tortuous vessels, i.e. vessels exhibiting complex geometry with tight bends.

It would be advantageous to discover alternative means of deploying mixing elements on a vascular catheter, preferably which are less bulky and more easily manufactured, but without compromising patient safety.

### SUMMARY OF THE PRESENT DISCLOSURE

According to a first aspect, there is provided a mixer sheath for a vascular catheter, the mixer sheath comprising a tube having a wall patterned with a line of weakness which is configured to cause buckling of a portion of the tube wall when a longitudinal compression force is applied to the tube to form a blood mixing element which extends radially outwards with respect to a location of the portion of the tube wall prior to buckling.

The line of weakness could be a cut line through the full thickness of the tube wall.

In an unbuckled state, the line of weakness could extend for at least some of its length in a direction with a non-zero longitudinal component with respect to an axis of the tube.

The line of weakness could be one of a pair of lines of weakness configured to cause buckling of the portion of the tube wall when a longitudinal compression force is applied to the tube such that part of the tube exterior surface extends radially outwards to form the mixing element, the mixing element comprising two diametrically opposed fins.

The lines of weakness forming the pair could at least partially overlap in their longitudinal extents.

In an unbuckled state, the lines of weakness forming the pair could be projections onto the tube of symmetrical images across a longitudinal line of symmetry on the tube surface.

The line of weakness could substantially form a longitudinally extending zigzag.

The line of weakness could change direction four times.

One or more sections of the zigzag could be curved such that the mixing element forms with a curved outer edge.

The minimum circumferential distance from one of the pair of lines of weakness to the other could be 0.3 mm.

The mixer sheath could comprise one or more further lines of weakness configured to cause buckling of a further portion of the tube wall when a longitudinal compression force is applied to the tube to form a further blood mixing element which extends radially outwards with respect to a location of the further portion of the tube wall prior to buckling.

The two or more mixing elements could together form a static mixer.

Longitudinally successive lines of weakness could be arranged at successive angular positions around a circumference of the tube so that, following buckling of the tube wall, longitudinally successive mixing elements extend at successive angles around the tube circumference.

Each successive angular position could be at ninety degrees to the last.

The line of weakness could vary in thickness along its length.

The mixer sheath could comprise a catheter connector configured to fix a distal end of the mixer sheath to a distal end of the catheter, such that the longitudinal compression force can be applied by moving a proximal end of the mixer sheath axially with respect to a proximal end of the catheter.

The tube wall could vary in thickness between one region of the mixer sheath and another.

The thickness of the tube wall at the locations of longitudinally successive lines of weakness could be successively greater so that, on application of the longitudinal compression force, longitudinally successive mixing elements form in a sequence corresponding to their longitudinal location.

The mixer sheath could comprise an inlet port for permitting blood flow through the tube wall.

The line of weakness could be configured such that, when the tube is buckled, the mixing element's outer edge extends radially to no more than 3 mm from the tube axis.

The tube wall could be no more than 0.5 mm thick.

The mixer sheath could be formed of polyether ether ketone (PEEK).

According to a second aspect, there is provided a method of manufacturing the mixer sheath of the first aspect, the method comprising: forming the tube; and forming the line of weakness using laser micromachining.

The method could comprise, after forming the tube, forming both of the pair of lines of weakness in a single operation by laser micromachining through both a side of the tube proximal to a laser performing the micromachining and a side distal to the laser.

The line of weakness could be formed prior to forming the tube, the tube being formed using seam welding and/or by encircling it with collars in two or more axially separated locations.

According to a third aspect, there is provided a catheter sheath comprising the mixer sheath of the first aspect and an outer sheath configured to at least partially enclose the mixer sheath.

According to a fourth aspect, there is provided a catheter system comprising the mixer sheath of the first aspect and a catheter, the mixer sheath being configured to at least partially enclose the catheter.

The catheter could be a blood sampling catheter comprising a lumen for withdrawing blood, the lumen being in fluid communication with the inlet port.

The mixer sheath could comprise one or more further inlet ports and the catheter could comprise one or more corresponding further lumens for withdrawing blood, each inlet port being in fluid communication with a respective lumen.

The catheter system could further comprise the outer sheath of the third aspect.

According to a fifth aspect, there is provided a method of deploying a vascular catheter, the method comprising: feeding the catheter system of the fourth aspect through a blood vessel to a desired location; and subsequently pushing on a proximal end of the mixer sheath while holding the catheter substantially stationary with respect to the vessel so as to apply the longitudinal compression force to the mixer sheath.

The method could further comprise, between the feeding and the pushing steps, withdrawing the outer sheath to expose a region of the mixer sheath in the vicinity of the line of weakness which is configured to buckle to form the mixing element.

The withdrawing step could only expose the portion of the tube wall which is configured to buckle to form one mixing element or one cluster of mixing elements, the method further comprising: subsequent to the pushing step, withdrawing the outer sheath further to expose the further portion of the tube wall which is configured to buckle to form the further mixing element; and subsequently pushing on the proximal end of the mixer sheath while holding the catheter substantially stationary with respect to the vessel so as to apply the longitudinal compression force to the mixer sheath again.

According to a sixth aspect, there is provided a method of removing a vascular catheter, the method comprising: with the catheter system of the fourth aspect deployed in a blood vessel, pulling on a proximal end of the mixer sheath while holding the catheter substantially stationary with respect to the vessel so as to apply a longitudinal extension force to the mixer sheath; and withdrawing the catheter system through the blood vessel.

The method could further comprise, between the pulling and the withdrawing steps, sliding the outer sheath over the mixer sheath towards its distal end.

According to a seventh aspect there is provided a method of deploying a blood mixing element, the method comprising pushing on a proximal end of the mixer sheath of the catheter system of the fourth aspect while holding the catheter substantially stationary so as to apply the longitudinal compression force to the mixer sheath.

The method can further comprise, before the pushing step, withdrawing the outer sheath to expose a region of the mixer sheath in the vicinity of the line of weakness which is configured to buckle to form the mixing element.

According to an eighth aspect there is provided a method of deploying blood mixing elements, the method comprising the method of the seventh aspect, wherein the withdrawing step only exposes the portion of the tube wall which is configured to buckle to form one mixing element or one cluster of mixing elements, the method further comprising: subsequent to the pushing step, withdrawing the outer sheath further to expose the further portion of the tube wall which is configured to buckle to form the further mixing element; and subsequently pushing on the proximal end of the mixer sheath while holding the catheter substantially stationary so as to apply the longitudinal compression force to the mixer sheath again.

According to a ninth aspect there is provided a method of using a catheter system, the method comprising the method of the seventh or eighth aspects, followed by sampling of blood withdrawn through the lumen.

According to a tenth aspect there is provided a method of reversing the deployment of a blood mixing element, the method taking place subsequent to the methods of any of the seventh to ninth aspects and comprising pulling on the proximal end of the mixer sheath of the catheter system of the fourth aspect while holding the catheter substantially stationary so as to apply a longitudinal extension force to the mixer sheath.

The method could further comprise, after the pulling step, sliding the outer sheath over the mixer sheath towards its distal end.

The method could further comprise the method of the ninth aspect.

### SUMMARY OF THE INVENTION

In accordance with the present invention, there is provided a mixer sheath for a vascular catheter device as defined in claim 1. In addition, further advantageous embodiments follow from the dependent claims.

Aspects of the present disclosure will now be described by way of example with reference to the accompanying figures. In the figures:
Figure 1 illustrates an example catheter system;
Figures 2A, 2B and 2C illustrate how an example catheter system like that of Figure 1 could be deployed in a blood vessel;
Figures 3A to 3E illustrate an example buckling process for the type of catheter system illustrated in Figures 1 and 2A to 2C;
Figures 4A and 4B show the pattern of a pair of lines of weakness on a flattened-out portion of an example mixer sheath;
Figure 5 shows how the pattern of Figure 4B could be repeated down the length of a mixer sheath;
Figure 6 illustrates an alternative example catheter system;
Figures 7A and 7B are flowcharts showing example methods for manufacturing a mixer sheath;
Figure 8A is a flowchart describing a method of deploying a vascular catheter with a mixer sheath; and
Figure 8B is a flowchart describing a method 850 of removing a vascular catheter.

The following description is presented to enable any person skilled in the art to make and use the system, and is provided in the context of a particular application. Various modifications to the disclosed embodiments will be readily apparent to those skilled in the art.

The terms "top", "bottom", "sides" and other terms describing the orientation of features are not intended to be limiting and are purely included in order to facilitate the description of the relative location of these features in the context of the accompanying drawings. In use, or during storage, the features may be disposed in other orientations.

In order to provide deployable external mixing elements on a vascular catheter, it is proposed to employ a "mixer sheath". The mixer sheath comprises a tube having a wall patterned with at least one line of weakness such that, when the tube is compressed longitudinally, the tube wall buckles into a configuration in which part of the tube wall extends radially outwards with respect to its prior location to form a blood mixing element. The blood mixing element is of a suitable size and shape to deflect a portion of blood flow which encounters it in use. It may for example be described as a fin, baffle, blade or vane.

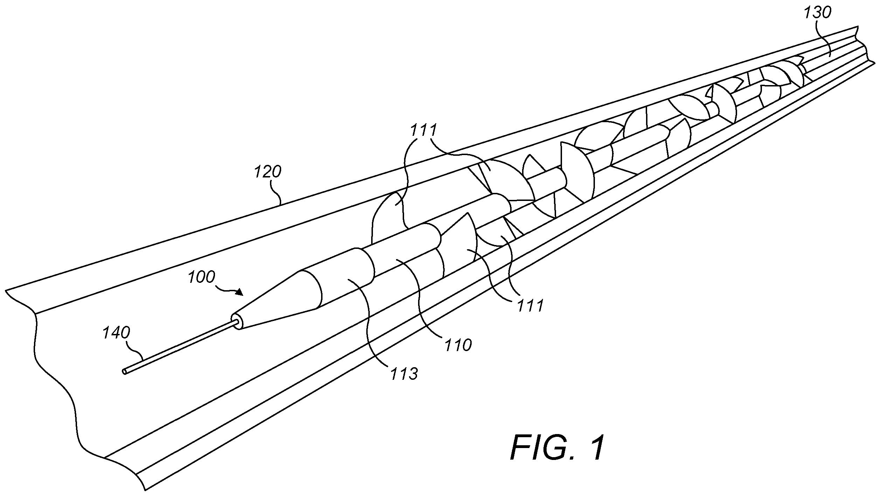

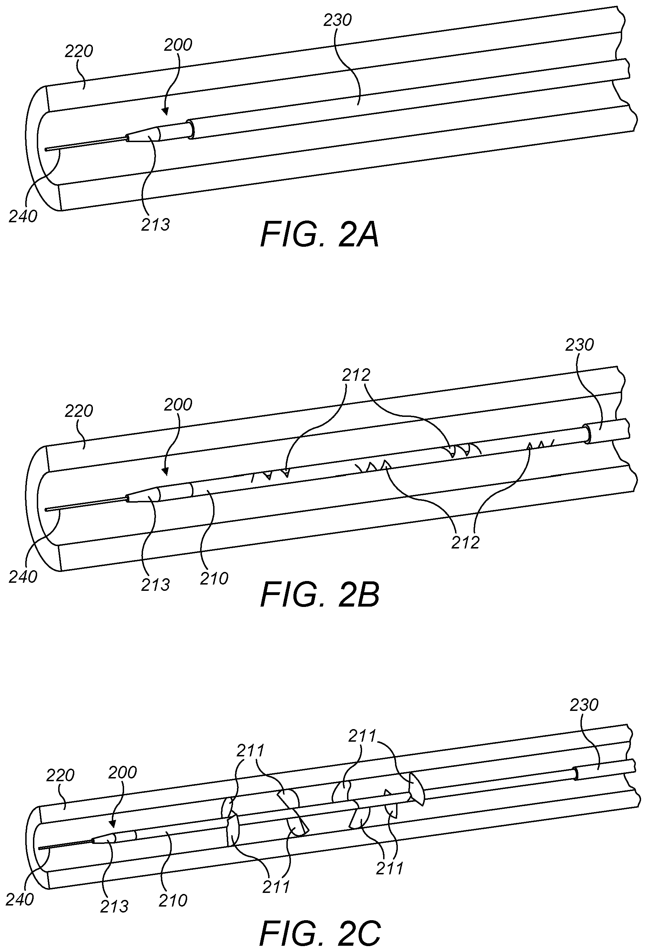

Figure 1 illustrates an example catheter system 100 having such a mixer sheath 110, with mixing elements 111 deployed in a blood vessel 120. The mixer sheath 110 comprises a tip 113 on its distal end. The tip 113 can comprise a catheter connector (not shown) which affixes the distal end of the mixer sheath 110 to the distal end of the catheter within the mixer sheath (not shown). (As used herein in relation to a catheter system, "distal" shall be taken to mean the free end, which traverses the longest distance through the patient's vasculature. Similarly, as used herein in relation to a catheter system, "proximal" shall be taken to mean the directly controlled end, which traverses the shortest distance through the patient's vasculature, and may not even enter the patient's vasculature at all.) An optional outer sheath 130 is shown, and will be described further in relation to Figures 2A to 2C. The catheter system can for example be designed with a central lumen to allow the catheter to be tracked over an interventional guide wire 140 until the required location of the catheter in the blood vessel 120 is achieved. The guide wire could also comprise radiopaque marker bands or fillers to allow precise location to be visualised, e.g. under fluoroscopic imaging.

In this example, the mixing elements occur in diametrically opposed pairs with a small axial offset between the two elements forming each pair, and a larger axial offset between adjacent pairs. Successive pairs of mixing elements are arranged at successive circumferential locations. In this case, the angular offset between successive pairs is ninety degrees, though other angular offsets could be employed. This type of arrangement creates a static mixer which mixes the blood through flow division (stratification) and radial mixing.

Figures 2A to 2C illustrate how an example catheter system like that of Figure 1 could be deployed in a blood vessel 220. First, the catheter system 200 is manoeuvred into position in the blood vessel 220 along a guide wire 240. As shown in Figure 2A, an outer sheath 230 could enclose some or all of the mixer sheath at this stage. (As shown, the catheter tip 213 is not enclosed by the outer sheath 230.) Next, as shown in Figure 2B, the outer sheath 230 is withdrawn to reveal the mixer sheath 210, in particular lines of weakness 212. Finally, the proximal end (not shown) of the mixer sheath 210 is pushed on while the central lumen of the catheter (joined to the tip, which in this example acts as a connector between the catheter and mixer sheath) within the mixer sheath is held stationary with respect to the blood vessel 220, causing longitudinal compression of the mixer sheath 210. This results in buckling of a portion of the mixer sheath 210 in the vicinity of each line of weakness 212 to produce mixer elements 211. (This result could be achieved in other ways, by any combination of pushing/pulling on the mixer sheath and/or the catheter that results in relative movement between them at one longitudinal location while they remain fixed relative to one another at another longitudinal location, the relative movement resulting in longitudinal compression of the mixer sheath.)

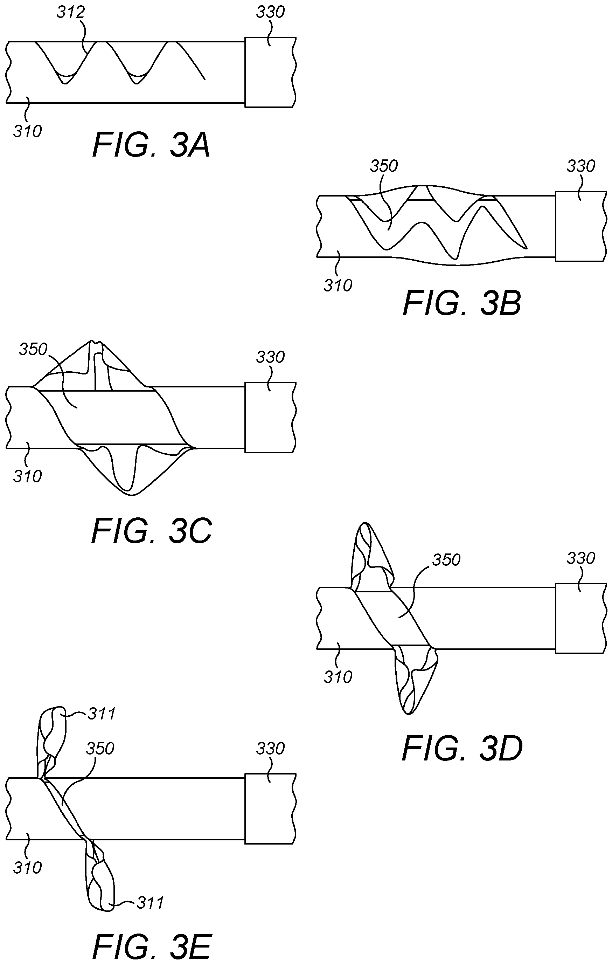

An example buckling process for the type of catheter system illustrated in Figures 1 and 2A to 2C is illustrated in Figures 3A to 3D. The mixer sheath 310 comprises a tube surrounding a catheter 350. Lines of weakness 312 are patterned on its external surface. In this case the lines of weakness 312 are cut lines all the way through the thickness of the tube. Figure 3A illustrates what this looks like following withdrawal of optional outer sheath 330. As the mixer sheath 310 is longitudinally compressed, the line of weakness 312 gapes to reveal the central tube of the catheter 350 within the mixer sheath 310, as shown in Figure 3B. With further longitudinal compression of the mixer sheath 310, the portions of the mixer sheath 310 around the gaping line of weakness 312 start to fold in on themselves as shown in Figure 3C. This folding/wrapping continues with further longitudinal compression of the mixer sheath 310, and the exposed region of the catheter 350 visible through the gaping line of weakness 312 becomes axially shorter as shown in Figure 3D, as the proximal portion of the mixer sheath 310 is pushed distally. This continues with further longitudinal compression of the mixer sheath 310 until the mixer elements 311 are fully formed as shown in Figure 3E, leaving only a thin strip of the catheter 350 visible between distal and proximal portions of the mixer sheath 310.

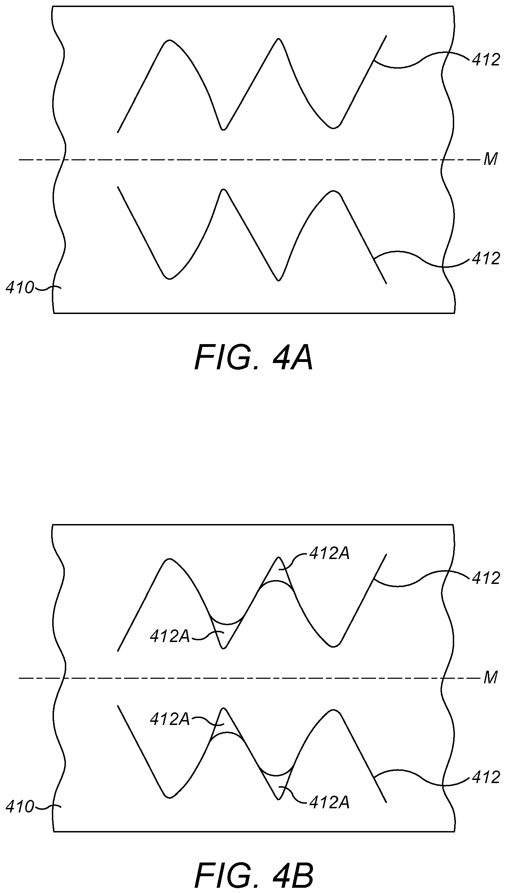

Although in Figures 3A to 3E only one line of weakness 312 is visible, for the buckling to work in the manner shown a pair of lines of weakness 412 in mixer sheath 410 is needed as shown in the examples of Figures 4A and 4B. (Alternatively, the pair of lines 412 could form a single line of weakness if they are connected, e.g. at their distal and/or proximal ends, by a portion of fold line, e.g. formed by scoring or perforating the mixer sheath.) Figures 4A and 4B show the pattern of this pair of lines of weakness on a flattened-out portion of the mixer sheath 410. Each line of weakness 412 forms a zigzag pattern which changes direction four times. In this flat view, the two lines of weakness 412 are reflections of one another in mirror line M. Thus, when this patterning is present on the mixer sheath in tube form, they are projections onto the tube of symmetrical images across a longitudinal line of symmetry on the tube surface. Figure 4B shows an example (which corresponds to the examples of Figures 1 to 3E) in which the lines of weakness 412 are thicker in some places than others, i.e. with the lines being slits in some parts and cut-outs in others. In particular, the lines are thicker towards the two central points of each zigzag pattern to form cut-outs 412a. This results in a rounded outer edge on the mixing elements, reducing the risk of them damaging the blood vessel wall. Making the lines of weakness thicker in some places, e.g. at their ends to make rounded corners, can also provide stress relief to minimise the risk of tears propagating.

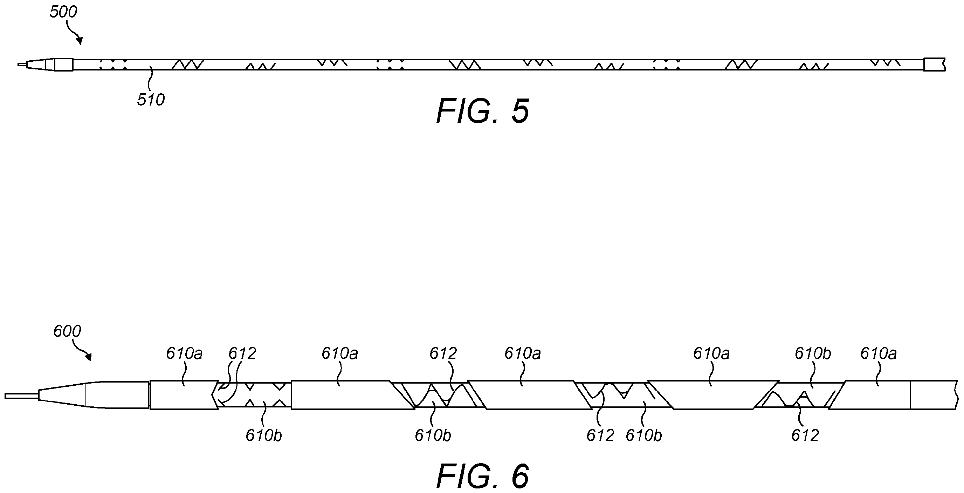

Figure 5 shows how the pattern of Figure 4B could be repeated down the length of a mixer sheath 510 to form a catheter system 500 capable of producing a static mixer that can substantially fully mix blood flow across the entire cross section of a blood vessel.

Figure 6 shows an alternative example catheter system 600 wherein the mixer sheath is reinforced in portions 610a not having lines of weakness 612 patterned thereon, relative to those portions 610b which do have the lines of weakness 612 patterned thereon, to encourage preferential buckling of the correct portions 610b, without allowing the system as a whole to become too flexible or weak. This could be achieved for example by forming the mixer sheath tube in laminated layers, and stripping one or more layers in the portions 610b having lines of weakness 612 patterned thereon. Alternatively the tube could be formed of a single layer, then etched/filed away in the portions 610b to have lines of weakness 612 patterned thereon. As another option, portions 610a not having lines of weakness 612 patterned thereon could be formed by sliding collars over a mixer sheath of the type shown in Figures 1 to 5, and optionally heat shrinking those collars or otherwise clamping them in position. Such collars could alternatively be formed by wrapping strips of tape around a mixer sheath of the type shown in Figures 1 to 5.

The thickness of the tube could be varied in any way desired to result in preferential buckling in some locations with respect to others. For example, a plurality of mixing elements could be configured to deploy in a particular sequence by making the tube thinnest in the region to buckle to form the first mixing element to be deployed, a little thicker in the region to buckle to form the second mixing element to be deployed, and so on to the final mixing element to be deployed which is formed in the thickest region of the tube (or the thickest buckling region, if other regions are made thicker for additional strength as described in relation to Figure 6.) If the sequence is defined according to longitudinal location, the tube wall could taper from one end to the other.

Although only a few examples have been described above in relation to figures 1 to 6, a buckling mixer sheath for a vascular catheter could take many forms. In general, it comprises a tube, which may be an entirely uniform open-ended cylinder, or may vary in composition and/or thickness and/or diameter and/or cross-sectional shape along its axial extent and/or around its cross-sectional perimeter (circumference). One or both of its ends may be closed.

The tube has a wall patterned with at least one line of weakness configured to cause buckling under the influence of a longitudinal (e.g. axial) compression force so as to produce at least one external blood mixing element. The line of weakness could be a continuous slit or cut-out through the entire thickness of the tube wall. The line could be formed of a train of slits or cut-outs, i.e. perforations. Alternatively it could be a continuous score line, i.e. a line along which the tube wall is thinner than the tube wall surrounding the line, whether this is achieved through moulding, additive manufacture processes, partial coextrusion/lamination or by removing material along the score line by e.g. mechanical or chemical methods. A non-continuous score line formed of a train of indentations could alternatively be used. The line of weakness could be formed of a combination of any two or more of the above types of lines, or in any other way that results in preferential buckling of a particular portion of the tube under longitudinal compression to produce a blood mixing element.

The line of weakness could extend substantially longitudinally when unbuckled, though as illustrated in Figures 4A and 4B the line need not be straight and can change direction one or more times.

Multiple mixing elements can be produced in clusters if multiple lines of weakness are located in a group, for example with at least some longitudinal overlap. For example, diametrically opposed mixing element "fins" can be produced as shown in Figures 3A to 3E if a pair of lines of weakness as illustrated in Figures 4A and 4B are employed. Other cluster configurations can be envisaged with different patterning of the lines of weakness, for example a tri- or quad-fin propeller type grouping. Fins of ach cluster could be arranged evenly around the circumference of the tube, or in an irregular formation. Alternatively, the patterning could be configured to produce single mixing elements rather than clusters.

As illustrated in Figures 4A and 4B, a zigzag pattern can be used to induce buckling. In that example the zigzag changes direction four times, but if the zigzag extended further, with additional changes of direction, the mixing elements formed would be thicker, and thus stronger.

The points of the zigzag could be rounded to give the mixing element a curved outer edge.

In the paired zigzag pattern illustrated in Figures 4A and 4B, the width of the stem of the mixing element is determined by the minimum circumferential distance between the two zigzags. This should be wide enough that the mixing elements cannot be torn off under the influence of the kinds of forces typically experienced by vessel wall contacting components of vascular catheter systems. The appropriate minimum width will thus depend on the material, construction and thickness of the mixer sheath. For example, for a mixer sheath formed of a single 0.5 mm thick sheet of polyether ether ketone (PEEK), a suitable minimum stem width is 0.3 mm. A suitable radial extent of the mixing element in this example could be up to 3 mm.

Other suitable materials for the mixer sheath include polyethylene terephthalate (PET), polyamide, polyimide and polytetrafluoroethylene (PTFE). The mixer sheath could be made of a single material, or could comprise components of multiple different materials, e.g. in a laminated structure.

If the catheter is a blood sampling catheter, then the mixer sheath can have one or more inlet ports so that blood can flow through the tube wall to the catheter. Each inlet pot could be in fluid communication with a lumen for removal of blood samples. The inlet ports could be configured such that the sliding of the mixer sheath with respect to the catheter to form the mixing elements could result in the ports aligning with corresponding lumen inlets, so that blood sampling only begins once the mixing elements are deployed. (The ports and lumen inlets could be configured so that they all align at the same instant, in a similar manner to the mechanism described in European patent number EP 1 912 556 B1.) Inlet ports on the mixer sheath could be shaped to exactly correspond to their respective lumen inlets, or could have a longer longitudinal extent to allow for slight variation in the extent of deployment of the mixing elements, for example if full deployment is prevented by an obstacle within the blood vessel such as a bend or constriction.

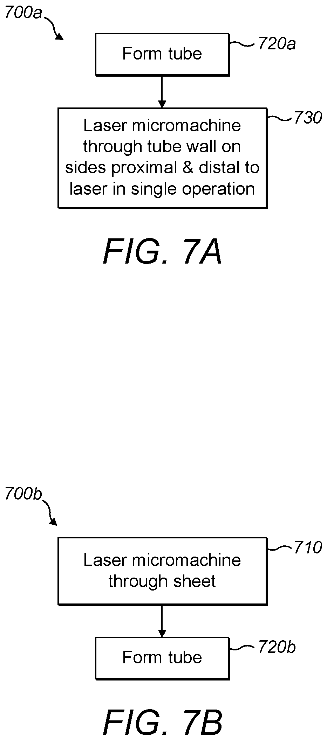

Figures 7A and 7B are flowcharts showing respective example methods 700a and 700b for manufacturing a mixer sheath such as the example mixer sheaths described above. Both methods use laser micromachining to form the line(s) of weakness precisely.

The method 700a of Figure 7A is to produce a mixer sheath having a pair of lines of weakness, for example as illustrated by Figures 4A and 4B. At step 720a the tube is formed by any suitable method. For example it could be extruded, produced by additive manufacturing technique (such as 3D printing) or constructed by connecting the long edges of a rectangular sheet together, e.g. by seam welding, with adhesive and/or by encircling the rolled sheet with collars in two or more locations along its length. If collars are used, they could also serve to strengthen the tube in regions in which buckling is not desired, as explained above in relation to Figure 6. A collar for holding the tube together could for example consist of a ring of elastic material, a ring of heat shrink which is slid into position and then shrunk to fit the tube, or a belt/strap which is tied around the tube with its two ends affixed together. At step 730, subsequent to forming the tube, a laser is aimed at the side of the tube and used to micromachine through the tube wall on the sides both near and far with respect to the laser at once. The laser could be aimed such that it describes a diameter of the tube's cross-section, or could be located off-centre, depending on the design of the mixing elements.

The method 700b of Figure 7B can be used for producing a mixer sheath with any configuration of one or more lines of weakness. At step 710 a line of weakness is patterned by laser micromachining a rectangular sheet of sheath material. Then, at step 720b the tube is formed from the sheet by connecting together the long edges of the sheet in any of the ways suggested above.

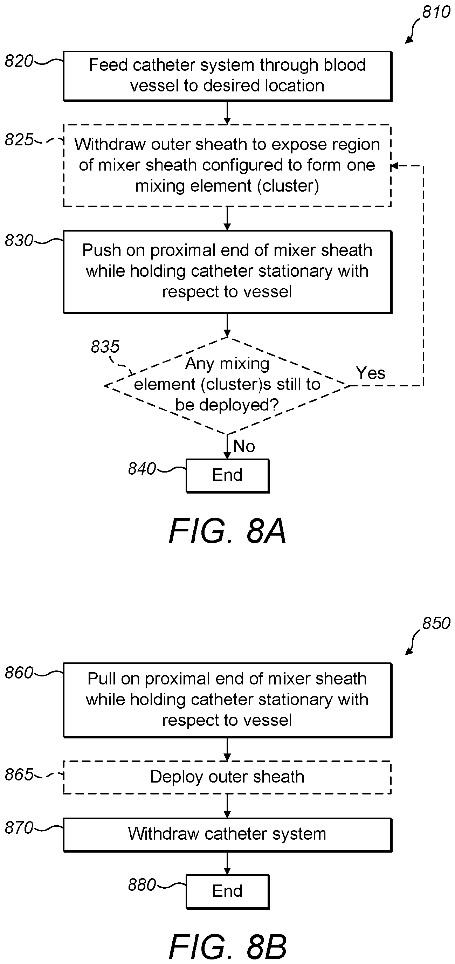

Figure 8A is a flowchart describing a method 810 of deploying a vascular catheter with a mixer sheath. At step 820, a catheter system is fed through a blood vessel to a desired location. Then, at step 830, the proximal end of the mixer sheath is pushed on while the catheter within is held substantially stationary, in order to deploy one or more mixing elements. The method 810 ends at 840.

If an outer sheath is used, then this must be withdrawn between steps 820 and 830 to allow room for formation of the mixing elements. For example, if a staged deployment is desired then at step 825 the outer sheath is withdrawn to expose a region of the mixer sheath configured to form one mixing element, or one mixing element cluster. Following step 830, if the result of querying at 835 whether any mixing elements or mixing element clusters remain to be deployed is positive, the flow returns to step 825. Otherwise, the method ends at 840.

Figure 8B is a flowchart describing a method 850 of removing a vascular catheter. At step 860 the proximal end of the mixer sheath is pulled while the catheter within is held stationary with respect to the blood vessel, in order to collapse the mixer elements. Then, at step 870 the catheter system is withdrawn from the vessel. The method ends at 880. If an outer sheath is used then this can be deployed between steps 860 and 880 by sliding it over the collapsed mixer sheath towards the distal end at 865.

Other embodiments will be apparent to those skilled in the art from consideration of the specification and practice of the embodiments disclosed herein. It is intended that the specification and examples be considered as exemplary only.

The invention is defined by the appended claims.

## Claims

1. A mixer sheath (210) for a vascular catheter, the mixer sheath comprising a tube having a wall patterned with a line of weakness (212) which is configured to cause buckling of a portion of the tube wall when a longitudinal compression force is applied to the tube to form a blood mixing element (211) which extends radially outwards with respect to a location of the portion of the tube wall prior to buckling, **characterized in that**, in an unbuckled state, the line of weakness extends for at least some of its length in a direction with a non-zero longitudinal component with respect to an axis of the tube.

2. The mixer sheath of claim 1, wherein the line of weakness is a cut line through the full thickness of the tube wall.

3. The mixer sheath of claim 1 or claim 2, wherein the line of weakness is one of a pair of lines of weakness configured to cause buckling of the portion of the tube wall when a longitudinal compression force is applied to the tube such that part of the tube exterior surface extends radially outwards to form the mixing element, the mixing element comprising two diametrically opposed fins.

4. The mixer sheath of claim 3, wherein the lines of weakness forming the pair at least partially overlap in their longitudinal extents.

5. The mixer sheath of any preceding claim, wherein the line of weakness substantially forms a longitudinally extending zigzag.

6. The mixer sheath of claim 5, wherein one or more sections of the zigzag are curved such that the mixing element forms with a curved outer edge.

7. The mixer sheath of any preceding claim, comprising one or more further lines of weakness configured to cause buckling of a further portion of the tube wall when a longitudinal compression force is applied to the tube to form a further blood mixing element which extends radially outwards with respect to a location of the further portion of the tube wall prior to buckling.

8. The mixer sheath of claim 7, wherein the two or more mixing elements together form a static mixer.

9. The mixer sheath of either of claims 7 or 8, wherein longitudinally successive lines of weakness are arranged at successive angular positions around a circumference of the tube so that, following buckling of the tube wall, longitudinally successive mixing elements extend at successive angles around the tube circumference.

10. The mixer sheath of claim 9 as dependent on claim 3, wherein each successive angular position is at ninety degrees to the last.

11. The mixer sheath of any preceding claim, wherein the line of weakness varies in thickness along its length.

12. The mixer sheath of any preceding claim, comprising a catheter connector configured to fix a distal end of the mixer sheath to a distal end of the catheter, such that the longitudinal compression force can be applied by moving a proximal end of the mixer sheath axially with respect to a proximal end of the catheter.

13. The mixer sheath of any preceding claim, wherein the tube wall varies in thickness between one region of the mixer sheath and another.

14. The mixer sheath of claim 13 as dependent directly or indirectly on claim 7, wherein the thickness of the tube wall at the locations of longitudinally successive lines of weakness is successively greater so that, on application of the longitudinal compression force, longitudinally successive mixing elements form in a sequence corresponding to their longitudinal location.

15. The mixer sheath of any preceding claim, comprising an inlet port for permitting blood flow through the tube wall.

## Patentansprüche

1. Mischer-Ummantelung (210) für einen Gefäßkatheter, wobei die Mischer-Ummantelung eine Röhre umfasst, die eine Wand aufweist, die mit einer Schwächungslinie (212) gemustert ist, die so konfiguriert ist, dass sie ein Knicken eines Abschnitts der Röhrenwand verursacht, wenn eine Längsdruckkraft auf die Röhre ausgeübt wird, um ein Blutmischelement (211), das sich hinsichtlich einer Lage des Abschnitts der Röhrenwand vor dem Knicken radial nach außen erstreckt, auszubilden,
**dadurch gekennzeichnet, dass**
sich die Schwächungslinie in einem nicht geknicktem Zustand über mindestens einen Teil ihrer Länge in einer Richtung mit einer von Null verschiedenen Längskomponente hinsichtlich einer Achse der Röhre erstreckt.

2. Mischer-Ummantelung nach Anspruch 1, wobei die Schwächungslinie eine Schnittlinie durch die gesamte Dicke der Röhrenwand ist.

3. Mischer-Ummantelung nach Anspruch 1 oder 2, wobei die Schwächungslinie eine eines Paares von Schwächungslinien ist, die so konfiguriert sind, dass sie ein Knicken des Abschnitts der Röhrenwand verursachen, wenn eine Längsdruckkraft derart auf die Röhre ausgeübt wird, dass sich ein Teil der Röhrenaußenoberfläche radial nach außen erstreckt, um das Mischelement auszubilden, wobei das Mischelement zwei diametral gegenüberliegende Rippen umfasst.

4. Mischer-Ummantelung nach Anspruch 3, wobei sich die das Paar ausbildenden Schwächungslinien in ihren Längsausdehnungen zumindest teilweise überlappen.

5. Mischer-Ummantelung nach einem der vorhergehenden Ansprüche, wobei die Schwächungslinie im Wesentlichen eine sich in Längsrichtung erstreckende Zickzacklinie ausbildet.

6. Mischer-Ummantelung nach Anspruch 5, wobei ein oder mehrere Bereiche der Zickzacklinie derart gekrümmt sind, dass sich das Mischelement mit einer gekrümmten Außenkante ausbildet.

7. Mischer-Ummantelung nach einem der vorhergehenden Ansprüche, die eine oder mehrere weitere Schwächungslinien umfasst, die so konfiguriert sind, dass sie ein Knicken eines weiteren Abschnitts der Röhrenwand verursachen, wenn eine Längsdruckkraft auf die Röhre ausgeübt wird, um ein weiteres Blutmischelement auszubilden, das sich hinsichtlich einer Lage des weiteren Abschnitts der Röhrenwand vor dem Knicken radial nach außen erstreckt.

8. Mischer-Ummantelung nach Anspruch 7, wobei die zwei oder mehr Mischelemente zusammen einen statischen Mischer ausbilden.

9. Mischer-Ummantelung nach Anspruch 7 oder 8, wobei in Längsrichtung aufeinanderfolgende Schwächungslinien an aufeinanderfolgenden Winkelpositionen um den Umfang der Röhrenwand herum angeordnet sind, so dass sich nach dem Knicken der Röhrenwand in Längsrichtung aufeinanderfolgende Mischelemente in aufeinanderfolgenden Winkeln um den Röhrenumfang herum erstrecken.

10. Mischer-Ummantelung nach Anspruch 9, wenn von Anspruch 3 abhängig, wobei jede aufeinanderfolgende Winkelposition bei neunzig Grad bis zur letzten liegt.

11. Mischer-Ummantelung nach einem der vorhergehenden Ansprüche, wobei die Schwächungslinie entlang ihrer Länge in der Dicke variiert.

12. Mischer-Ummantelung nach einem der vorhergehenden Ansprüche, die einen Katheterverbinder umfasst, der so konfiguriert ist, dass er ein distales Ende der Mischer-Ummantelung an einem distalen Ende des Katheters derart fixiert, dass die Längsdruckkraft durch axiales Bewegen eines proximalen Endes der Mischer-Ummantelung hinsichtlich eines proximalen Endes des Katheters ausgeübt werden kann.

13. Mischer-Ummantelung nach einem der vorhergehenden Ansprüche, wobei die Dicke der Röhrenwand zwischen einem Bereich der Mischer-Ummantelung und einem anderen variiert.

14. Mischer-Ummantelung nach Anspruch 13, wenn direkt oder indirekt von Anspruch 7 abhängig, wobei die Dicke der Röhrenwand an den Lagen von in Längsrichtung aufeinanderfolgenden Schwächungslinien aufeinanderfolgend größer wird, so dass sich bei Ausübung der Längsdruckkraft in Längsrichtung aufeinanderfolgende Mischelemente in einer Reihenfolge, die ihrer Lage in Längsrichtung entspricht, ausbilden

15. Mischer-Ummantelung nach einem der vorhergehenden Ansprüche, die eine Einlassöffnung umfasst, um den Blutfluss durch die Röhrenwand zu ermöglichen.

## Revendications

1. Gaine de mélangeur (210) destinée à un cathéter vasculaire, la gaine de mélangeur comprenant un tube ayant une paroi qui présente un motif de ligne de faiblesse (212), laquelle ligne est conçue pour provoquer la déformation d'une portion de la paroi du tube lorsqu'une force de compression longitudinale est appliquée au tube pour former un élément de mélange de sang (211) qui s'étend radialement vers l'extérieur par rapport à un emplacement de la portion de la paroi du tube avant la déformation,
**caractérisée en ce que**, dans un état non déformé, la ligne de faiblesse s'étend, sur au moins une partie de sa longueur, dans une direction avec un composant longitudinal non nul par rapport à un axe du tube.

2. Gaine de mélangeur selon la revendication 1, dans laquelle la ligne de faiblesse est une ligne de coupe traversant toute l'épaisseur de la paroi du tube.

3. Gaine de mélangeur selon la revendication 1 ou 2, dans laquelle la ligne de faiblesse est l'une d'une paire de lignes de faiblesse conçue pour provoquer la déformation de la portion de la paroi du tube lorsqu'une force de compression longitudinale est appliquée au tube de telle sorte que cette partie de la surface extérieure du tube s'étend radialement vers l'extérieur pour former l'élément de mélange, l'élément de mélange comprenant deux ailettes diamétralement opposées.

4. Gaine de mélangeur selon la revendication 3, dans laquelle les lignes de faiblesse formant la paire se chevauchent au moins partiellement dans leurs étendues longitudinales.

5. Gaine de mélangeur selon l'une des revendications précédentes, dans laquelle la ligne de faiblesse forme sensiblement un zigzag s'étendant longitudinalement.

6. Gaine de mélangeur selon la revendication 5, dans laquelle une ou plusieurs sections du zigzag sont courbées de telle sorte que l'élément de mélange se forme avec un bord extérieur courbé.

7. Gaine de mélangeur selon l'une des revendications précédentes, comprenant une ou plusieurs autres lignes de faiblesse conçues pour provoquer la déformation d'une autre portion de la paroi du tube lorsqu'une force de compression longitudinale est appliquée au tube pour former un autre élément de mélange de sang qui s'étend radialement vers l'extérieur par rapport à un emplacement de l'autre portion de la paroi du tube avant la déformation.

8. Gaine de mélangeur selon la revendication 7, dans laquelle les deux ou plusieurs éléments de mélange forment ensemble un mélangeur statique.

9. Gaine de mélangeur selon l'une des revendications 7 ou 8, dans laquelle des lignes de faiblesse successives dans le sens longitudinal sont disposées à des positions angulaires successives autour d'une circonférence du tube de sorte que, après la déformation de la paroi du tube, des éléments de mélange successifs dans le sens longitudinal s'étendent à des angles successifs autour de la circonférence du tube.

10. Gaine de mélangeur selon la revendication 9 lorsqu'elle dépend de la revendication 3, dans laquelle chaque position angulaire successive est à quatre-vingt-dix degrés par rapport à la dernière.

11. Gaine de mélangeur selon l'une des revendications précédentes, dans laquelle la ligne de faiblesse varie en épaisseur sur sa longueur.

12. Gaine de mélangeur selon l'une des revendications précédentes, comprenant un raccord de cathéter conçu pour fixer une extrémité distale de la gaine de mélangeur à une extrémité distale du cathéter, de telle sorte que la force de compression longitudinale peut être appliquée en déplaçant une extrémité proximale de la gaine de mélangeur axialement par rapport à une extrémité proximale du cathéter.

13. Gaine de mélangeur selon l'une des revendications précédentes, dans laquelle la paroi du tube varie en épaisseur entre une région de la gaine de mélangeur et une autre.

14. Gaine de mélangeur selon la revendication 13 lorsqu'elle dépend directement ou indirectement de la revendication 7, dans laquelle l'épaisseur de la paroi du tube aux emplacements des lignes de faiblesse successives dans le sens longitudinal augmente progressivement de sorte que, lors de l'application de la force de compression longitudinale, des éléments de mélange successifs dans le sens longitudinal forment une séquence correspondant à leur emplacement longitudinal.

15. Gaine de mélangeur selon l'une des revendications précédentes, comprenant un orifice d'entrée pour permettre l'écoulement du sang à travers la paroi du tube.
